# EUROPÄISCHE PATENTSCHRIFT

(11) **EP 4 217 012 B1**
(45) Veröffentlichungstag und Bekanntmachung des Hinweises auf die Patenterteilung: **26.02.2025**
(21) Anmeldenummer: 21783206.2
(22) Anmeldetag: 23.09.2021
(51) Int. Cl.: A61L 9/16, B64D 13/06, B08B 7/00, B64F 5/30, B64F 5/40

(54) **VERFAHREN ZUR WARTUNG EINER FLUGZEUGKLIMAANLAGE UND FLUGZEUGKLIMAANLAGENSYSTEM**
METHOD OF MAINTAINING AN AIRCRAFT AIR CONDITIONING SYSTEM
PROCÉDURE D'ENTRETIEN D'UN SYSTÈME DE CLIMATISATION D'AVION

(30) Priorität: 28.09.2020 DE 102020125202
(43) Veröffentlichungstag der Anmeldung: 02.08.2023
(73) Patentinhaber: Airbus Operations GmbH, 21129 Hamburg (DE)
(72) Erfinder: CHEN, Paul, 21129 Hamburg (DE); KOENIG, Markus, 21129 Hamburg (DE); BAUMANN, Joerg, 21129 Hamburg (DE); KAUFHOLD, Michael, 21129 Hamburg (DE)
(74) Vertreter: Schicker, Silvia
(86) Internationale Anmeldenummer: PCT/EP2021/076217
(87) Internationale Veröffentlichungsnummer: WO 2022/063906

(56) Entgegenhaltungen:
- CN-A- 111 268 141
- US-B2- 9 526 806
- ZHI SUN ET AL: "A Novel Aircraft Air Conditioning System with a Sterilization Unit by Ultra@BULLETHigh@BULLETTemperature Air Stream", TRANSACTIONS OF NANJING UNIVERSITY OF AERONAUTICS AND ASTRONAUTICS, 31 August 2020 (2020-08-31), pages 1 - 9, XP055868674, Retrieved from the Internet <URL:https://kns.cnki.net/kcms/detail/detail.aspx?doi=10.16356/j.1005-1120.2020.04.015> [retrieved on 20211202], DOI: 10.16356/j.1005-1120.2020.04.015
- YAP TE FAYE ET AL: "A predictive model of the temperature-dependent inactivation of coronaviruses", APPLIED PHYSICS LETTERS, AMERICAN INSTITUTE OF PHYSICS, 2 HUNTINGTON QUADRANGLE, MELVILLE, NY 11747, vol. 117, no. 6, 11 August 2020 (2020-08-11), XP012249287, ISSN: 0003-6951, [retrieved on 20200811], DOI: 10.1063/5.0020782

## Beschreibung

Die Erfindung betrifft ein Verfahren zur Wartung einer Flugzeugklimaanlage sowie ein Flugzeugkli maanlagensystem.

An Bord eines Flugzeugs, insbesondere eines Passagierflugzeugs sollte das Auftreten schlechter Gerüche möglichst vermieden werden. Eine Reduktion schlechter Gerüche ist üblicherweise durch eine Verringerung des Wassergehalts in einem Luftverteilungssystem des Flugzeugs möglich, da trockene Luft üblicherweise als "frischer" wahrgenommen wird als feuchte Luft. Darüber hinaus kann es erforderlich sein, unerwünschte Substanzen, wie z.B. Bakterien, Viren oder dergleichen aus dem Luftverteilungssystem des Flugzeugs zu entfernen.

Aus der US 9,526,806 B2 ist ein Verfahren und System zu Dekontamination von Flugzeugabteilen bekannt. Entsprechend erkannten Verunreinigungen werden einzuspritzendes Desinfektionsmittel, Luftstrom, Lufttemperatur und Luftfeuchtigkeit gewählt, um die Flugzeugabteile dann zu dekontaminieren.

Aus dem Artikel mit dem Titel "A Novel Aircraft Air Conditioning System with a Sterilization Unit by Ultra-High-Temperature Air Stream" veröffentlicht in dem Magazin "Transactions of Nanjing University of Aeronautics and Astronautics", Band 37, Nummer 4, ist es bekannt einen Hochtemperaturluftstrom zur Dekontamination einer Rezirkulationsluftmenge in einer Bypass-Leitung einer Flugzeugklimaanlage zu nutzen, bevor die Rezirkulationsluft abgekühlt und dann wieder als Teil der klimatisierten Mischluft der Flugzeugkabine zugeführt wird.

Der Erfindung liegt die Aufgabe zugrunde, ein Verfahren zur Wartung einer Flugzeugklimaanlage sowie ein Flugzeugklimaanlagensystem bereitzustellen, die eine kontrollierte Dekontamination eines Luftverteilungssystems eines Flugzeugs zur Beseitigung schlechter Gerüche und/oder unerwünschter Substanzen ermöglichen.

Diese Aufgabe wird durch ein Verfahren zur Wartung einer Flugzeugklimaanlage mit den Merkmalen des Patentanspruchs 1 und ein Flugzeugklimaanlagensystem mit den Merkmalen des Patentanspruchs 7 gelöst.

Bei einem Verfahren zur Wartung einer Flugzeugklimaanlage wird ein Wartungssteuergerät mit einem Steuersystem der Flugzeugklimaanlage verbunden. Bei dem Wartungssteuergerät handelt es sich vorzugsweise um ein Steuergerät, das im Bodenbetrieb des Flugzeugs von einer Wartungscrew temporär mit dem Steuersystem der Flugzeugklimaanlage verbunden wird. Beispielsweise kann das Wartungssteuergerät in Form eines tragbaren Steuergeräts ausgebildet sein, das von der Wartungscrew an Bord des Flugzeugs gebracht wird. Alternativ dazu ist es jedoch auch denkbar, ein an Bord des Flugzeugs vorgesehenes Steuergerät als Wartungssteuergerät einzusetzen, das beispielsweise im Bodenbetrieb des Flugzeugs von der Wartungscrew aktiviert werden kann.

Im nächsten Schritt wird ein von dem Wartungssteuergerät ausgegebenes Dekontaminations-Aktivierungssignal zur Aktivierung eines Dekontaminationsprozesses an das Steuersystem der Flugzeugklimaanlage übertragen. Das Steuersystem der Flugzeugklimaanlage kann eine Mehrzahl von Steuergeräten zur Steuerung des Betriebs der verschiedenen Bereiche der Flugzeugklimaanlage umfassen. Beispielsweise kann das Steuersystem der Flugzeugklimaanlage ein Zapfluftsystem-Steuergerät, ein Pack-Steuergerät und ein zentrales Zonen-Steuergerät umfassen.

Das Zapfluftsystem-Steuergerät kann dazu vorgesehen sein, den Betrieb eines Zapfluftsystems der Flugzeugklimaanlage und insbesondere die Zufuhr von Zapfluft von einem Triebwerk oder einem Hilfstriebwerk des Flugzeugs zu der Flugzeugklimaanlage zu steuern. Das Pack-Steuergerät ist vorzugweise dazu eingerichtet, den Betrieb eines Klimapacks der Flugzeugklimaanlage zu steuern. Das zentrale Zonen-Steuergerät ist vorzugsweise dazu eingerichtet, die Zufuhr von entsprechend temperierter Klimatisationsluft in die einzelnen Klimazonen einer Flugzeugkabine zu steuern. Beispielsweise kann das zentrale Zonen-Steuergerät dazu eingerichtet sein, den Betrieb von Klimaanlagenkomponenten, wie z.B. Ventilen, Gebläsen, etc. zu steuern, die einem Klimatisationsluftzufuhrsystem der Flugzeugklimaanlage zugeordnet sind. Alle Steuergeräte des Steuersystems der Flugzeugklimaanlage sind vorzugsweise mit einem Flight Warning System verbunden, das dazu eingerichtet ist, im Fall einer Fehlfunktion der Flugzeugklimaanlage ein entsprechendes Warnsignal auszugeben. Ferner kann eine Statusanzeige vorgesehen sein, die ggf. anzeigt, dass eine Dekontamination zumindest eines Teilbereichs eines Luftverteilungssystems des Flugzeugs durchgeführt wird.

Die Ausgabe des Dekontaminations-Aktivierungssignals durch das Wartungssteuergerät kann manuell initiiert werden, beispielsweise wenn während eines vorangegangenen Fluges oder durch die Wartungscrew im Bodenbetrieb des Flugzeugs festgestellt wird, dass eine Dekontamination zumindest eines Teilbereichs eines Luftverteilungssystems des Flugzeugs gewünscht oder erforderlich ist, um schlechte Gerüche und/oder unerwünschter Substanzen zu beseitigen. Alternativ oder zusätzlich dazu ist es jedoch auch denkbar, dass das Wartungssteuergerät das Dekontaminations-Aktivierungssignal automatisch ausgibt, beispielsweise wenn das Wartungssteuergerät von einem entsprechenden Messgerät oder einem Steuergerät des Flugzeugs ein Signal erhält, das anzeigt, dass eine Dekontamination des Luftverteilungssystems des Flugzeugs gewünscht oder erforderlich ist. Der zu dekontaminierende Teilbereich des Luftverteilungssystems kann ebenfalls manuell durch die Wartungscrew oder automatisch in Reaktion auf den Empfang eines entsprechenden Signals ausgewählt werden, das von einem Messgerät oder einem Steuergerät an das Wartungssteuergerät ausgegeben wird.

In Reaktion auf den Empfang des Dekontaminations-Aktivierungssignals steuert das Steuersystem der Flugzeugklimaanlage den Betrieb der Flugzeugklimaanlage derart, dass zumindest ein zu dekontaminierender Teilbereich des Luftverteilungssystems eines mit der Flugzeugklimaanlage ausgestatteten Flugzeugs auf eine Dekontaminationstemperatur aufgeheizt wird, die höher ist als eine Betriebstemperatur des Teilbereichs des Luftverteilungssystems im Normalbetrieb der Flugzeugklimaanlage. Durch das Aufheizen auf die Dekontaminationstemperatur wird der Wasserdampfpartialdruck in dem der Dekontaminationstemperatur ausgesetzten Bereich des Luftverteilungssystems erhöht und folglich der Wassergehalt in diesem Bereich des Luftverteilungssystems reduziert. Dadurch können unangenehme Gerüche beseitigt werden. Darüber hinaus werden durch die erhöhten Temperaturen unerwünschte Substanzen, wie z.B. Bakterien, Viren oder dergleichen aus dem Luftverteilungssystem entfernt.

Das erfindungsgemäße Verfahren zur Wartung einer Flugzeugklimaanlage ermöglicht eine automatische kontrollierte Dekontamination des Luftverteilungssystems der Flugzeugklimaanlage, die auch unter schwierigen Umgebungsbedingungen, z.B. kalten Umgebungstemperaturen, in effizienter Art und Weise unter der Steuerung des Steuersystems der Flugzeugklimaanlage erfolgt. Auf den zeitintensiven Einsatz von Simulations-Tools, die durch die Umgehung der Sensoren und Steuergeräte der Flugzeugklimaanlage eine manuelle Temperaturerhöhung in dem Luftverteilungssystem der Flugzeugklimaanlage erlauben, kann daher in vorteilhafter Weise verzichtet werden. Darüber hinaus wird eine Überhitzung einzelner Teilbereiche und/oder Komponenten des Luftverteilungssystems zuverlässig verhindert.

Vorzugsweise steuert das Steuersystem der Flugzeugklimaanlage in Reaktion auf den Empfang des Dekontaminations-Aktivierungssignals den Betrieb der Flugzeugklimaanlage derart, dass in dem zu dekontaminierenden Teilbereich des Luftverteilungssystems die Dekontaminationstemperatur für eine definierte Zeitspanne beibehalten wird. Die Zeitspanne wird vorzugsweise durch das von dem Wartungssteuergerät ausgegebene Dekontaminations-Aktivierungssignal vorgegeben und kann an die jeweilige Dekontaminationsaufgabe angepasst sein. Beispielsweise kann eine längere Zeitspanne für die Aufrechterhaltung der Dekontaminationstemperatur vorgesehen werden, wenn festgestellt wird, dass in dem zu dekontaminierenden Teilbereich des Luftverteilungssystems eine große Wassermenge enthalten ist oder wenn gezielt bestimmte Bakterien und/oder Viren aus dem zu dekontaminierenden Teilbereich des Luftverteilungssystems entfernt werden sollen.

Zusätzlich oder alternativ dazu kann das Steuersystem der Flugzeugklimaanlage den Betrieb der Flugzeugklimaanlage zur Dekontamination zumindest eines Teilbereichs des Luftverteilungssystems in Abhängigkeit von mindestens einem Umgebungsparameter steuern. Ein wesentlicher Umgebungsparameter, der vorzugsweise bei der Steuerung des Betriebs der Flugzeugklimaanlage zur Dekontamination zumindest eines Teilbereichs des Luftverteilungssystems berücksichtigt wird, ist beispielsweise die Umgebungstemperatur.

Beispielsweise kann es bei kalten Umgebungstemperaturen schwierig sein, einen Kompressor der Flugzeugklimaanlage für sich genommen so anzusteuern, dass der Kompressor auf die gewünschte Dekontaminationstemperatur aufgeheizt wird. Bei kalten Umgebungstemperaturen kann das Steuersystem der Flugzeugklimaanlage jedoch auf diese erschwerten Bedingungen reagieren, indem es beispielsweise den Betrieb von dem Kompressor vorgeschalteten Komponenten so steuert, dass dem Kompressor bereits wärmere Luft zugeführt wird und der Kompressor somit auf die gewünschte Dekontaminationstemperatur erwärmt werden kann.

Zur Aktivierung einer Dekontamination eines Zapfluftsystems des Luftverteilungssystems gibt das Wartungssteuergerät ein Zapfluftsystem-Dekontaminations-Aktivierungssignal an das Steuersystem der Flugzeugklimaanlage aus. In Reaktion auf den Empfang des Zapfluftsystem-Dekontaminations-Aktivierungssignals steuert das Steuersystem der Flugzeugklimaanlage den Betrieb eines Zapfluftsystems der Flugzeugklimaanlage dann derart, dass das Zapfluftsystem des Luftverteilungssystems auf eine Dekontaminationstemperatur von ca. 220 bis 240° C aufgeheizt wird, die höher ist als die Betriebstemperatur des Zapfluftsystems im Normalbetrieb der Flugzeugklimaanlage. Dadurch wird eine gezielte, gesteuerte Dekontamination des Zapfluftsystems des Luftverteilungssystems ermöglicht. Das Zapfluftsystem umfasst vorzugsweise mindestens eine ein Triebwerk und/oder ein Hilfstriebwerk des Flugzeugs mit einem Klimapack, insbesondere einem Primärwärmetauscher des Klimapacks verbindende Zapfluftleitung sowie mit dieser Leitung verbundene Komponenten, wie z.B. Ventile oder dergleichen. Ferner kann das Zapfluftsystem einen Zapflufttemperatursensor zur Überwachung der Temperatur der dem Klimapack zugeführten Zapfluft umfassen.

Die Dekontamination des Zapfluftsystems wird vorzugsweise von dem Zapfluftsystem-Steuergerät des Steuersystems der Flugzeugklimaanlage gesteuert, das im Normalbetrieb der Flugzeugklimaanlage dazu vorgesehen ist, die Zufuhr von Zapfluft von einem Triebwerk oder einem Hilfstriebwerk des Flugzeugs zu der Flugzeugklimaanlage zu steuern. Dementsprechend hat das Zapfluftsystem-Steuergerät Zugriff auf alle wesentlichen zu steuernden Komponenten des Zapfluftsystems und kann daher alle Steuerungseingriffe vornehmen, die erforderlich sind, um das Zapfluftsystems auf die Dekontaminationstemperatur aufzuheizen.

Vorzugsweise wird das Zapfluftsystems des Luftverteilungssystems auf eine Dekontaminationstemperatur von ca. 230 °C aufgeheizt. Bei dieser Temperatur ist eine ordnungsgemäße Dekontamination des Zapfluftsystems gewährleistet. Gleichzeitig wird eine Überhitzung von in dem Zapfluftsystem des Luftverteilungssystems verbauten Komponenten zuverlässig verhindert.

Ein mit einer Zapfluftleitung verbundener Primärwärmetauscher der Flugzeugklimaanlage kann wahlweise in einen Prozess zur Dekontamination des Zapfluftsystems einbezogen oder von diesem Prozess ausgenommen werden. Beispielsweise kann der Primärwärmetauscher in den Prozess zur Dekontamination des Zapfluftsystems einbezogen werden, wenn der Primärwärmetauscher ebenfalls dekontaminiert werden soll und/oder wenn einem stromabwärts des Primärwärmetauschers angeordneten Kompressor Luft mit einer erhöhten Temperatur zugeführt werden soll. Dies kann beispielsweise erwünscht oder erforderlich sein, wenn der Kompressor ebenfalls dekontaminiert werden soll, bei kalten Umgebungstemperaturen jedoch für sich genommen nicht auf die gewünschte Dekontaminationstemperatur erhitzt werden kann. Um den Primärwärmetauscher in den Prozess zur Dekontamination des Zapfluftsystems einzubeziehen oder von diesem Prozess auszunehmen, kann ein stromaufwärts des Wärmetauschers in der Zapfluftleitung angeordnetes Zapfluft-Steuerventil, beispielsweise von dem Zapfluftsystem-Steuergerät, entsprechend gesteuert werden.

Zur Aktivierung einer Dekontamination eines Kompressorbereichs des Luftverteilungssystems gibt das Wartungssteuergerät ein Kompressorbereich-Dekontaminations-Aktivierungssignal an das Steuersystem der Flugzeugklimaanlage aus. In Reaktion auf den Empfang des Kompressorbereich-Dekontaminations-Aktivierungssignals steuert das Steuersystem der Flugzeugklimaanlage den Betrieb eines Kompressors der Flugzeugklimaanlage dann derart, dass der Kompressorbereich des Luftverteilungssystems auf eine Dekontaminationstemperatur von ca. 210 bis 230° C aufgeheizt wird, die höher ist als die Betriebstemperatur des Kompressorbereichs im Normalbetrieb der Flugzeugklimaanlage. Dadurch wird eine gezielte, gesteuerte Dekontamination des Kompressorbereichs des Luftverteilungssystems ermöglicht. Der Kompressorbereich umfasst vorzugsweise einen Kompressor, der eine Komponente eines Klimapacks der Flugzeugklimaanlage bildet. Ferner können dem Kompressorbereich stromabwärts des Kompressors angeordnete Komponenten der Flugzeugklimaanlage zugeordnet sein.

Die Dekontamination des Kompressorbereichs wird vorzugsweise von dem Pack-Steuergerät des Steuersystems der Flugzeugklimaanlage gesteuert, das im Normalbetrieb der Flugzeugklimaanlage dazu vorgesehen ist, den Betrieb der in dem Kompressorbereich vorgesehenen Komponenten der Flugzeugklimaanlage und insbesondere der Komponenten des Klimapacks der Flugzeugklimaanlage zu steuern. Dementsprechend hat das Pack-Steuergerät Zugriff auf alle wesentlichen zu steuernden Komponenten, die in dem Kompressorbereich verbaut sind und kann daher alle Steuerungseingriffe vornehmen, die erforderlich sind, um den Kompressorbereich auf die Dekontaminationstemperatur aufzuheizen.

Vorzugsweise wird der Kompressorbereich des Luftverteilungssystems auf eine Dekontaminationstemperatur von ca. 220 °C aufgeheizt. Bei dieser Temperatur ist eine ordnungsgemäße Dekontamination des Kompressorbereichs gewährleistet. Gleichzeitig wird eine Überhitzung von in dem Kompressorbereich des Luftverteilungssystems verbauten Komponenten zuverlässig verhindert.

Vorzugsweise umfasst der Kompressorbereich einen mit einem Auslass des Kompressors verbundenen Hauptwärmetauscher der Flugzeugklimaanlage. Dementsprechend kann der Hauptwärmetauscher in vorteilhafter Weise in einen Prozess zur Dekontamination des Kompressorbereichs einbezogen und ebenfalls dekontaminiert werden.

Ferner können dem Kompressorbereich auch weitere stromabwärts des Kompressors bzw. des Hauptwärmetauschers angeordnete Komponenten der Flugzeugklimaanlage, wie z.B. ein Nachheizer, ein Kondensator, eine Turbine sowie entsprechende Leitungen und Ventile zugeordnet sein.

Zur Aktivierung einer Dekontamination eines Klimatisationsluftzufuhrsystems des Luftverteilungssystems kann das Wartungssteuergerät ein Klimatisationsluftzufuhrsystem-Dekontaminations-Aktivierungssignal an das Steuersystem der Flugzeugklimaanlage ausgeben. In Reaktion auf den Empfang des Klimatisationsluftzufuhrsystem-Dekontaminations-Aktivierungssignals kann das Steuersystem der Flugzeugklimaanlage den Betrieb eines mit dem Klimatisationsluftzufuhrsystem des Luftverteilungssystems verbundenen Klimapacks der Flugzeugklimaanlage, den Betrieb mindestens eines in dem Klimatisationsluftzufuhrsystem des Luftverteilungssystems angeordneten Trimmluftventils und/oder den Betrieb mindestens eines in dem Klimatisationsluftzufuhrsystem des Luftverteilungssystems vorgesehenen Rezirkulationsluftgebläses derart steuert, dass das Klimatisationsluftzufuhrsystem des Luftverteilungssystems auf eine Dekontaminationstemperatur aufgeheizt wird, die höher ist als die Betriebstemperatur des Klimatisationsluftzufuhrsystems im Normalbetrieb der Flugzeugklimaanlage. Dadurch wird eine gezielte, gesteuerte Dekontamination des Klimatisationsluftzufuhrsystems des Luftverteilungssystems ermöglicht.

Das Klimatisationsluftzufuhrsystem umfasst vorzugsweise mindestens eine Leitung, die ein Klimapack der Flugzeugklimaanlage mit einem zu klimatisierenden Bereich einer Flugzeugkabine verbindet. Ferner können dem Klimatisationsluftzufuhrsystem Komponenten der Flugzeugklimaanlage zugeordnet sein, die den Klimatisationsluftstrom von einem Klimapack in einen zu klimatisierenden Bereich der Flugzeugkabine steuern, beispielsweise entsprechende Gebläse, Ventile und dergleichen. Eine Mischkammer, in der die von dem Klimapack bereitgestellte Klimatisationsluft mit aus der Flugzeugkabine abgeführter Rezirkulationsluft gemischt wird, mindestens ein Rezirkulationsgebläse, mindestens eine Rezirkulationsluftleitung, die einen zu klimatisierenden Bereich der Flugzeugkabine mit der Mischkammer verbindet, ein Trimluftventil sowie eine Trimluftleitung, die dazu eingerichtet ist, Trimluft in die Mischkammer zu leiten, können ebenfalls dem Klimatisationsluftzufuhrsystems zugeordnet sein.

Die Dekontamination des Klimatisationsluftzufuhrsystems wird vorzugsweise von dem Pack-Steuergerät und/oder dem zentralen Zonen-Steuergerät des Steuersystems der Flugzeugklimaanlage gesteuert. Durch die Einbeziehung des Pack-Steuergeräts in einen Prozess zur Dekontamination des Klimatisationsluftzufuhrsystems kann die Temperatur der Luft am Auslass des Klimapacks vor dem Eintritt in das Klimatisationsluftzufuhrsystem wie gewünscht gesteuert werden. Insbesondere kann Temperatur der Luft am Auslass des Klimapacks erhöht werden, um das Klimatisationsluftzufuhrsystem auf die gewünschte erhöhte Dekontaminationstemperatur aufzuheizen. Das zentrale Zonen-Steuergerät ist im Normalbetrieb der Flugzeugklimaanlage dazu vorgesehen, den Betrieb der in dem Klimatisationsluftzufuhrsystem vorgesehenen Komponenten der Flugzeugklimaanlage, insbesondere Ventilen und Gebläsen zu steuern, die die Zufuhr von Klimatisationsluft und Rezirkulationsluft in die Mischkammer der Flugzeugklimaanlage und weiter in einen zu klimatisierenden Bereich der Flugzeugkabine ermöglichen. Durch eine entsprechende Ansteuerung des Trimluftventils, kann bei Bedarf warme Trimluft zugeführt werden, um das Klimatisationsluftzufuhrsystem auf die gewünschte erhöhte Dekontaminationstemperatur aufzuheizen.

Vorzugsweise wird das Klimatisationsluftzufuhrsystem des Luftverteilungssystems auf eine Dekontaminationstemperatur von ca. 60 bis 80 °C, insbesondere von ca. 70 °C aufgeheizt. Bei dieser Temperatur ist eine ordnungsgemäße Dekontamination des Klimatisationsluftzufuhrsystems gewährleistet. Gleichzeitig wird eine Überhitzung von in dem Klimatisationsluftzufuhrsystem des Luftverteilungssystems verbauten Komponenten verhindert.

Die Dekontaminationstemperatur in dem Klimatisationsluftzufuhrsystem des Luftverteilungssystems wird vorzugsweise in Abhängigkeit einer zulässigen Maximaltemperatur von in einen Avionikbereich der Flugzeugkabine geleiteter Klimatisationsluft bestimmt. Dadurch wird eine Überhitzung von in dem Avionikbereich verbauten elektronischen Komponenten verhindert.

Ein Flugzeugklimaanlagensystem umfasst eine Flugzeugklimaanlage mit einem Steuersystem. Ferner umfasst das Flugzeugklimaanlagensystem ein Wartungssteuergerät, das mit dem Steuersystem der Flugzeugklimaanlage verbindbar und dazu eingerichtet ist, ein Dekontaminations-Aktivierungssignal zur Aktivierung eines Dekontaminationsprozesses auszugeben und an das Steuersystem der Flugzeugklimaanlage zu übertragen. Das Steuersystem der Flugzeugklimaanlage ist dazu eingerichtet, in Reaktion auf den Empfang des Dekontaminations-Aktivierungssignals den Betrieb der Flugzeugklimaanlage derart zu steuern, dass zumindest ein zu dekontaminierender Teilbereich eines Luftverteilungssystems eines mit der Flugzeugklimaanlage ausgestatteten Flugzeugs auf eine Dekontaminationstemperatur aufgeheizt wird, die höher ist als eine Betriebstemperatur des Teilbereichs des Luftverteilungssystems im Normalbetrieb der Flugzeugklimaanlage.

Das Steuersystem der Flugzeugklimaanlage ist vorzugsweise dazu eingerichtet, den Betrieb der Flugzeugklimaanlage derart zu steuern, dass in dem zu dekontaminierenden Teilbereich des Luftverteilungssystems die Dekontaminationstemperatur für eine definierte Zeitspanne beibehalten wird. Ferner kann das Steuersystem der Flugzeugklimaanlage dazu eingerichtet sein, den Betrieb der Flugzeugklimaanlage zur Dekontamination zumindest eines Teilbereichs des Luftverteilungssystems in Abhängigkeit von mindestens einem Umgebungsparameter, insbesondere in Abhängigkeit von einer Umgebungstemperatur zu steuern. Darüber hinaus kann das Steuersystem der Flugzeugklimaanlage dazu eingerichtet sein, den Betrieb der Flugzeugklimaanlage zur Dekontamination zumindest eines Teilbereichs des Luftverteilungssystems in Abhängigkeit eines Betriebszustands mindestens eines Rezirkulationsluftgebläses und/oder in Abhängigkeit eines Betriebszustands eines Trimluftsystems, insbesondere mindestens eines Trimluftventils zu steuern. Das Ziel dabei ist, eine möglichst hohe Temperatur auch zur Aufheizung der Kabine und des Cockpits zu erzeugen, insbesondere um Viren und/oder Bakterien zu eliminieren, ohne dabei die erlaubten Temperaturen in einer Avionicluftzuleitung zu überschreiten.

Das Wartungssteuergerät ist vorzugsweise dazu eingerichtet, zur Aktivierung einer Dekontamination eines Zapfluftsystems des Luftverteilungssystems ein Zapfluftsystem-Dekontaminations-Aktivierungssignal an das Steuersystem der Flugzeugklimaanlage auszugeben. Das Steuersystem der Flugzeugklimaanlage kann dazu eingerichtet sein, in Reaktion auf den Empfang des Zapfluftsystem-Dekontaminations-Aktivierungssignals den Betrieb eines Zapfluftsystems der Flugzeugklimaanlage derart zu steuern, dass das Zapfluftsystem des Luftverteilungssystems auf eine Dekontaminationstemperatur aufgeheizt wird, die höher ist als die Betriebstemperatur des Zapfluftsystems im Normalbetrieb der Flugzeugklimaanlage.

Das Steuersystem der Flugzeugklimaanlage kann ein Zapfluftsystem-Steuergerät umfassen, das dazu eingerichtet ist, die Dekontamination des Zapfluftsystems zu steuern. Ferner kann das Steuersystem der Flugzeugklimaanlage ein zentrales Zonen-Steuergerät umfassen. Das zentrale Zonen-Steuergerät kann dazu eingerichtet sein, das von dem Wartungssteuergerät ausgegebene Zapfluftsystem-Dekontaminations-Aktivierungssignal an das Zapfluftsystem-Steuergerät weiterzuleiten. Die Dekontaminationstemperatur in dem Zapfluftsystem des Luftverteilungssystems beträgt vorzugsweise ca. 220 bis 240 °C, insbesondere ca. 230 °C. Das Zapfluftsystem-Steuergerät kann dazu eingerichtet sein, einen mit einer Zapfluftleitung verbundenen Primärwärmetauscher der Flugzeugklimaanlage wahlweise in einen Prozess zur Dekontamination des Zapfluftsystems einzubeziehen oder von diesem Prozess auszunehmen. Um dies zu erreichen kann das Zapfluftsystem-Steuergerät dazu eingerichtet sein, ein stromaufwärts des Primärwärmetauschers in der Zapfluftleitung angeordnetes Zapfluft-Steuerventil entsprechend zu steuern.

Das Wartungssteuergerät ist vorzugsweise ferner dazu eingerichtet, zur Aktivierung einer Dekontamination eines Kompressorbereichs des Luftverteilungssystems ein Kompressorbereich-Dekontaminations-Aktivierungssignal an das Steuersystem der kann Flugzeugklimaanlage auszugeben. Das Steuersystem der Flugzeugklimaanlage dazu eingerichtet sein, in Reaktion auf den Empfang des Kompressorbereich-Dekontaminations-Aktivierungssignals den Betrieb eines Kompressors der Flugzeugklimaanlage derart zu steuern, dass der Kompressorbereich des Luftverteilungssystems auf eine Dekontaminationstemperatur aufgeheizt wird, die höher ist als die Betriebstemperatur des Kompressorbereichs im Normalbetrieb der Flugzeugklimaanlage.

Das Steuersystem der Flugzeugklimaanlage kann ein Pack-Steuergerät umfassen, das dazu eingerichtet ist, die Dekontamination des Kompressorbereichs zu steuern. Das zentrale Zonen-Steuergerät kann dazu eingerichtet sein, das von dem Wartungssteuergerät ausgegebene Kompressorbereich-Dekontaminations-Aktivierungssignal an das Pack-Steuergerät weiterzuleiten. Die Dekontaminationstemperatur in dem Kompressorbereich des Luftverteilungssystems beträgt vorzugsweise ca. 210 bis 230 °C, insbesondere ca. 220 °C. Der Kompressorbereich umfasst vorzugsweise einen mit einem Auslass des Kompressors verbundenen Hauptwärmetauscher der Flugzeugklimaanlage.

Das Wartungssteuergerät kann ferner dazu eingerichtet sein, zur Aktivierung einer Dekontamination eines Klimatisationsluftzufuhrsystems des Luftverteilungssystems ein Klimatisationsluftzufuhrsystem-Dekontaminations-Aktivierungssignal an das Steuersystem der Flugzeugklimaanlage auszugeben. Das Steuersystem der Flugzeugklimaanlage kann dazu eingerichtet sein, in Reaktion auf den Empfang des Klimatisationsluftzufuhrsystem-Dekontaminations-Aktivierungssignals den Betrieb eines mit dem Klimatisationsluftzufuhrsystem des Luftverteilungssystems verbundenen Klimapacks der Flugzeugklimaanlage, den Betrieb mindestens eines in dem Klimatisationsluftzufuhrsystem des Luftverteilungssystems angeordneten Trimmluftventils und/oder den Betrieb mindestens eines in dem Klimatisationsluftzufuhrsystem des Luftverteilungssystems angeordneten Rezirkulationsluftgebläses derart zu steuern, dass das Klimatisationsluftzufuhrsystem des Luftverteilungssystems auf eine Dekontaminationstemperatur aufgeheizt wird, die höher ist als die Betriebstemperatur des Klimatisationsluftzufuhrsystems im Normalbetrieb der Flugzeugklimaanlage.

Das zentrale Zonen-Steuergerät und/oder das Pack-Steuergerät des Steuersystems der Flugzeugklimaanlage kann/können dazu eingerichtet sein, die Dekontamination des Klimatisationsluftzufuhrsystems zu steuern. Das zentrale Zonen-Steuergerät kann dazu eingerichtet sein, das von dem Wartungssteuergerät ausgegebene Klimatisationsluftzufuhrsystem-Dekontaminations-Aktivierungssignal an das Pack-Steuergerät weiterzuleiten. Die Dekontaminationstemperatur in dem Kompressorbereich des Luftverteilungssystems beträgt vorzugsweise ca. 60 bis 80 °C, insbesondere ca. 70 °C. Das zentrale Zonen-Steuergerät und/oder das Pack-Steuergerät ist/sind vorzugsweise dazu eingerichtet, die Dekontaminationstemperatur in dem Klimatisationsluftzufuhrsystem des Luftverteilungssystems in Abhängigkeit einer zulässigen Maximaltemperatur von in einen Avionikbereich geleiteter Klimatisationsluft zu bestimmen.

Eine bevorzugte Ausführungsform der Erfindung wird nun anhand der beigefügten schematischen Zeichnungen näher erläutert, von denen
- Figur 1: ein Flugzeugklimaanlagensystem mit einer schematisch angedeuteten Flugzeugklimaanlage und einem Wartungssteuergerät zeigt, wobei das Wartungssteuergerät mit einem Steuersystem der Flugzeugklimaanlage verbunden ist;
- Figur 2: ein Zapfluftsystem eines Luftverteilungssystems der Flugzeugklimaanlage zeigt;
- Figur 3: einen Kompressorbereich eines Luftverteilungssystems der Flugzeugklimaanlage zeigt; und
- Figur 4: ein Klimatisationsluftzufuhrsystem der Flugzeugklimaanlage zeigt.

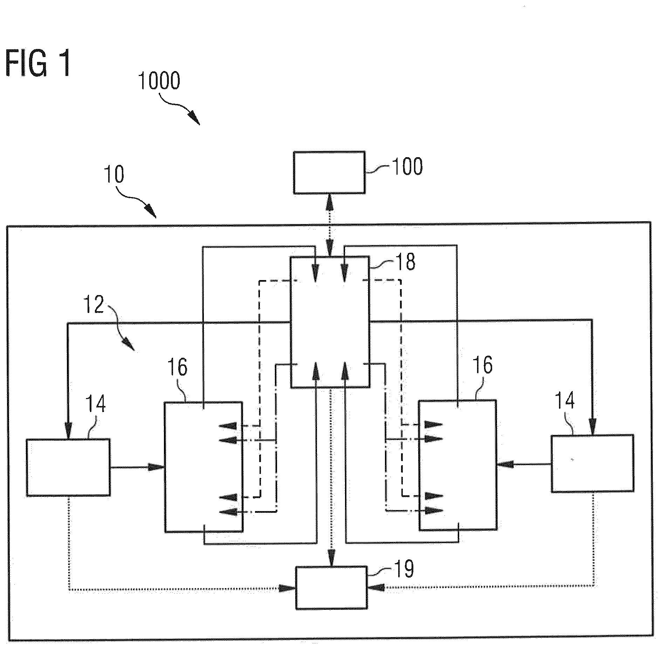

Figur 1 zeigt ein Flugzeugklimaanlagensystem 1000, das eine der in Figur 1 lediglich schematisch angedeutete Flugzeugklimaanlage 10 sowie ein Wartungssteuergerät 100 umfasst. In seiner hier gezeigten Ausführungsform ist das Wartungssteuergerät 100 in Form eines tragbaren Steuergeräts ausgebildet, das im Bodenbetrieb eines mit der Flugzeuglimaanlage 10 ausgestatteten Flugzeugs von einer Wartungscrew an Bord des Flugzeugs gebracht und temporär mit einem Steuersystem 12 der Flugzeugklimaanlage 10 verbunden wird. Alternativ dazu kann auch ein internes Wartungsinterface benutzt werden.

Das Steuersystem 12 der Flugzeugklimaanlage 10 umfasst zwei Zapfluftsystem-Steuergeräte 14, zwei Pack-Steuergeräte 16 und ein zentrales Zonen-Steuergerät 18. Alle Steuergeräte 14, 16, 18 des Steuersystems 12 der Flugzeugklimaanlage 10 sind mit einem Flight Warning System 19 verbunden, das dazu eingerichtet ist, im Fall einer Fehlfunktion der Flugzeugklimaanlage 10 ein entsprechendes Warnsignal auszugeben.

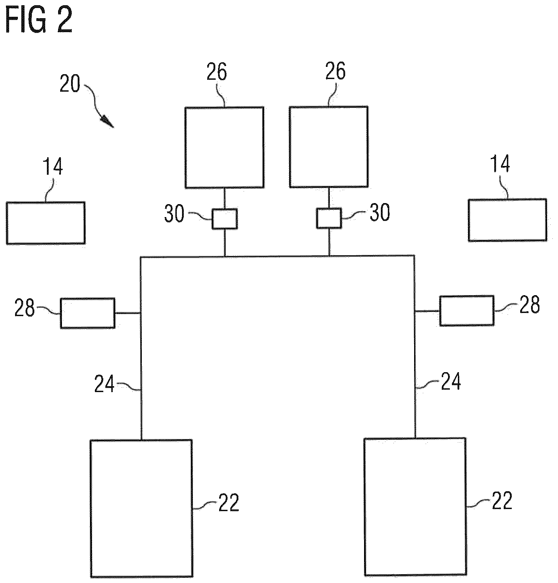

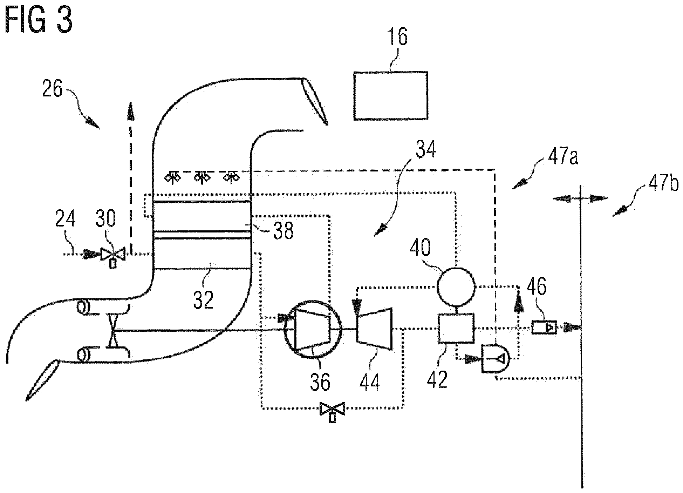

Im Normalbetrieb der Flugzeugklimaanlage 10 steuern die Zapfluftsystem-Steuergeräte 14 den Betrieb eines in Figur 2 schematisch veranschaulichten Zapfluftsystems 20 der Flugzeugklimaanlage 10 und insbesondere die Zufuhr von Zapfluft von einem Triebwerk 22 oder einem Hilfstriebwerk des Flugzeugs zu der Flugzeugklimaanlage 10. Das Zapfluftsystem 20 umfasst zwei Zapfluftleitungen 24, die jeweils ein Triebwerk 22 mit einem Klimapack 26 verbinden. Ferner sind in dem Zapfluftsystem 20 in Figur 3 nicht gezeigte Komponenten, wie z.B. Zapfluft-Zweigleitungen zum Abzapfen von Zapfluft an unterschiedlichen Stellen des Triebwerks 22 sowie Ventile und dergleichen zur Steuerung des Zapfluftstroms durch die Zapfluftleitungen 24 vorgesehen.

Jeder der Zapfluftleitungen 24 ist ein Zapflufttemperatursensor 28 zur Überwachung der Temperatur der dem entsprechenden Klimapack 26 zugeführten Zapfluft zugeordnet. Von den Zapflufttemperatursensoren 28 ausgegebene Signale, die die Temperatur der die entsprechende Zapfluftleitung 24 durchströmende Zapfluft anzeigen, werden an das entsprechenden Zapfluftsystem-Steuergerät 14 übertragen. Ferner ist in jeder der Zapfluftleitungen 24 ein Zapfluft-Steuerventil 30 vorgesehen, das unter der Steuerung des entsprechenden Pack-Steuergeräts 16 betrieben wird, um die Zufuhr von Zapfluft zu einem Klimapack 26 zu steuern.

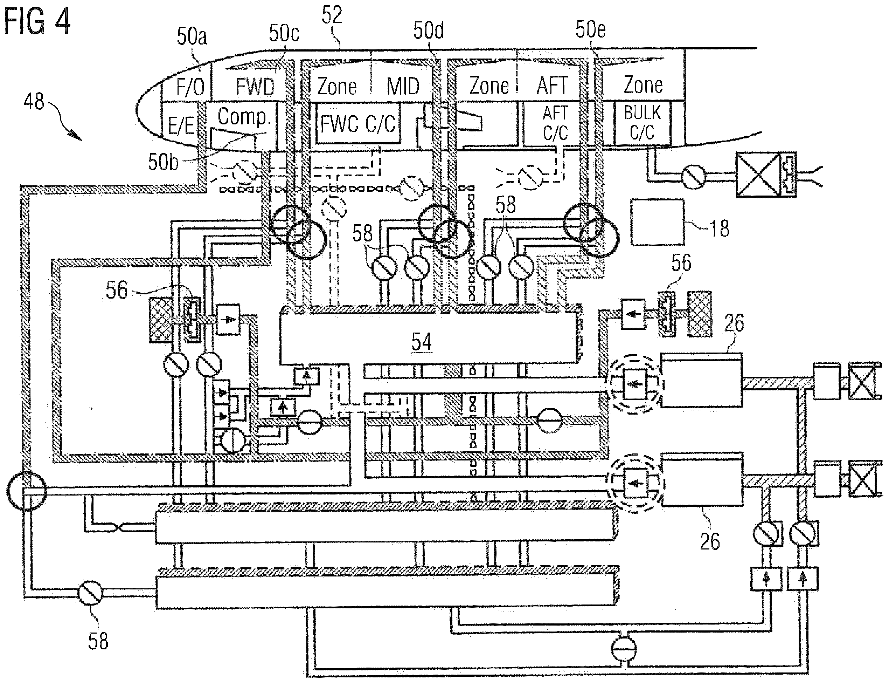

Jedes Pack-Steuergerät 16 steuert im Normalbetrieb der Flugzeugklimaanlage 10 den Betrieb eines in Figur 3 detaillierter veranschaulichten Klimapacks 26 der Flugzeugklimaanlage 10. Jedes Klimapack 26 umfasst einen mit einer Zapfluftleitung 24 verbundenen Primärwärmetauscher 32 sowie einen stromabwärts des Primärwärmetauschers 32 angeordneten Kompressorbereich 34. Dem Kompressorbereich 34 sind ein Kompressor 36, ein mit einem Auslass des Kompressors 36 verbundener Hauptwärmetauscher 38 sowie weitere stromabwärts des Kompressors 36 bzw. des Hauptwärmetauschers 38 angeordnete Komponenten, insbesondere ein Nachheizer 40, ein Kondensator 42, eine Turbine 44 sowie entsprechende Leitungen und Ventile zugeordnet. Ein Pack-Ventil 46 ist in Form eines Rückschlagentils ausgebildet und verhindert die Rückströmung von Klimatisationsluft aus einem bedruckten Bereich 47b des Flugzeugs in einen unbedruckten Bereich 47a des Flugzeugs. In dem bedruckten Bereich 47b des Flugzeugs ist ein in Figur 4 gezeigtes Klimatisationsluftzufuhrsystem 48 installiert.

Das Klimatisationsluftzufuhrsystem 48 umfasst eine Mehrzahl von Leitungen, die die Klimapacks 26 mit entsprechenden Klimazonen 50a, 50b, 50c, 50d, 50e einer Flugzeugkabine 52 verbinden. Ferner sind dem Klimatisationsluftzufuhrsystem 48 Komponenten der Flugzeugklimaanlage 10 zugeordnet, die den Klimatisationsluftstrom von einem Klimapack 26 in eine Klimazone 50a, 50b, 50c, 50d, 50e der Flugzeugkabine 52 steuern, insbesondere entsprechende Gebläse, Ventile und dergleichen. Eine Mischkammer 54, in der die von den Klimapacks 26 bereitgestellte Klimatisationsluft mit aus der Flugzeugkabine 52 abgeführter Rezirkulationsluft gemischt wird, Rezirkulationsgebläse 56, Rezirkulationsluftleitungen, Trimluftventile 58 sowie eine Trimluftleitung, die dazu dient, heiße Trimluft in die Mischkammer 54 zu leiten, können ebenfalls dem Klimatisationsluftzufuhrsystem 48 zugeordnet sein.

Im Normalbetrieb der Flugzeugklimaanlage 10 steuert das zentrale Zonen-Steuergerät 18 die Zufuhr von entsprechend temperierter Klimatisationsluft in die einzelnen Klimazonen 50a, 50b, 50c, 50d, 50e. Insbesondere steuert das zentrale Zonen-Steuergerät 18 den Betrieb der in dem Klimatisationsluftzufuhrsystem 48 vorgesehenen Ventile, Gebläse, etc.

Bei einem Verfahren zur Wartung der Flugzeugklimaanlage 10 wird das Wartungssteuergerät 100 mit dem Steuersystem 12 der Flugzeugklimaanlage 10 verbunden. Wenn die Wartungscrew feststellt, dass eine Dekontamination zumindest eines Teilbereichs eines durch das das Zapfluftsystems 20, den Kompressorbereich 34 und das Klimatisationsluftzufuhrsystem 48 gebildeten Luftverteilungssystems des Flugzeugs gewünscht oder erforderlich ist, um schlechte Gerüche und/oder unerwünschter Substanzen zu beseitigen, wird von der Wartungscrew die Ausgabe eines Dekontaminations-Aktivierungssignals zur Aktivierung eines Dekontaminationsprozesses initiiert. Dabei kann die Wartungscrew in einem von dem Wartungssteuergerät 100 bereitgestellten Auswahlmenü auswählen, welcher Teilbereich des Luftverteilungssystems dekontaminiert werden soll. Das von dem Wartungssteuergerät 100 ausgegebene Dekontaminations-Aktivierungssignal wird an das Steuersystem 12 der Flugzeugklimaanlage 10 übertragen.

In Reaktion auf den Empfang des Dekontaminations-Aktivierungssignals steuert das Steuersystem 12 der Flugzeugklimaanlage 10 den Betrieb der Flugzeugklimaanlage 10 derart, dass zumindest ein zu dekontaminierender Teilbereich des Luftverteilungssystems auf eine Dekontaminationstemperatur aufgeheizt wird, die höher ist als eine Betriebstemperatur des Teilbereichs des Luftverteilungssystems im Normalbetrieb der Flugzeugklimaanlage 10. Dadurch wird der Wassergehalt in diesem Bereich des Luftverteilungssystems reduziert, wodurch unangenehme Gerüche beseitigt werden. Darüber hinaus werden durch die erhöhten Temperaturen unerwünschte Substanzen, wie z.B. Bakterien, Viren oder dergleichen aus dem Luftverteilungssystem entfernt. Ferner das Flight Warning System auf einem Systemdisplay eine Statusmeldung an, dass eine Dekontamination zumindest eines Teilbereichs des Luftverteilungssystems des Flugzeugs durchgeführt wird.

Das Steuersystem 12 der Flugzeugklimaanlage 10 steuert in Reaktion auf den Empfang des Dekontaminations-Aktivierungssignals den Betrieb der Flugzeugklimaanlage 10 insbesondere derart, dass in dem zu dekontaminierenden Teilbereich des Luftverteilungsystems die Dekontaminationstemperatur für eine definierte Zeitspanne beibehalten wird. Diese Zeitspanne wird durch das von dem Wartungssteuergerät 100 ausgegebene Dekontaminations-Aktivierungssignal vorgegeben und ist an die jeweilige Dekontaminationsaufgabe angepasst. Insbesondere wird eine längere Zeitspanne für die Aufrechterhaltung der Dekontaminationstemperatur vorgesehen werden, wenn in dem zu dekontaminierenden Teilbereich des Luftverteilungsystems eine große Wassermenge enthalten ist oder wenn gezielt bestimmte Bakterien und/oder Viren aus dem zu dekontaminierenden Teilbereich des Luftverteilungsystems entfernt werden sollen.

Zusätzlich dazu steuert das Steuersystem 12 der Flugzeugklimaanlage 10 den Betrieb der Flugzeugklimaanlage 10 zur Dekontamination zumindest eines Teilbereichs des Luftverteilungssystems in Abhängigkeit von mindestens einem Umgebungsparameter, insbesondere in Abhängigkeit der Umgebungstemperatur. Darüber hinaus steuert das Steuersystem 12 der Flugzeugklimaanlage 10 den Betrieb der Flugzeugklimaanlage 10 zur Dekontamination zumindest eines Teilbereichs des Luftverteilungssystems in Abhängigkeit des Betriebszustands mindestens eines Rezirkulationsluftgebläses 56 und/oder in Abhängigkeit des Betriebszustands des Trimluftsystems, insbesondere mindestens eines Trimluftventils 58, um eine möglichst hohe Temperatur zur Aufheizung der Kabine 52 und des Cockpits zu erzeugen, ohne dabei die erlaubten Temperaturen in einer Avionicluftzuleitung zu überschreiten.

Wenn eine Dekontamination des Zapfluftsystems 20 des Luftverteilungssystems durchgeführt werden soll, wählt die Wartungscrew in dem Auswahlmenü des Wartungssteuergeräts 100 einen entsprechenden Menüpunkt "Dekontamination Zapfluftsystem", woraufhin das Wartungssteuergerät 100 ein Zapfluftsystem-Dekontaminations-Aktivierungssignal an das Steuersystem 12 der Flugzeugklimaanlage 10 ausgibt. Die Ausgabe des Zapfluftsystem-Dekontaminations-Aktivierungssignals erfolgt an das zentrale Zonen-Steuergerät 18, das seinerseits das Signal an eines der Zapfluftsystem-Steuergeräte 14 oder beide Zapfluftsystem-Steuergeräte 14 weiterleitet.

In Reaktion auf den Empfang des Zapfluftsystem-Dekontaminations-Aktivierungssignals steuert das Steuersystem 12 der Flugzeugklimaanlage 10, d. h. eines der Zapfluftsystem-Steuergeräte 14 oder beide Zapfluftsystem-Steuergeräte 14, den Betrieb des Zapfluftsystems 20 dann derart, dass das Zapfluftsystem 20 auf eine Dekontaminationstemperatur aufgeheizt wird, die höher ist als die Betriebstemperatur des Zapfluftsystems 20 im Normalbetrieb der Flugzeugklimaanlage 10. Dies erfolgt beispielsweise dadurch, dass dem Zapfluftsystem 20 Zapfluft zugeführt wird, die eine höhere Temperatur aufweist als im Normalbetrieb des Zapfluftsystems 20. Insbesondere wird der Zapfluftbereich 20 auf eine Dekontaminationstemperatur von ca. 220 bis 240 °C, vorzugsweise von ca. 230 °C aufgeheizt. Im Normalbetrieb wird das Zapfluftsystem 20 dagegen in der Regel bei einer Temperatur von ca. 200 °C betrieben. Eine Überwachung der Temperatur in dem Zapfluftsystem 20 erfolgt mithilfe der Zapflufttemperatursensoren 28.

Ein mit der Zapfluftleitung 24 verbundener Primärwärmetauscher 32 eines Klimapacks 26 kann wahlweise in einen Prozess zur Dekontamination des Zapfluftsystems 20 einbezogen oder von diesem Prozess ausgenommen werden. Beispielsweise kann der Primärwärmetauscher 32 in den Prozess zur Dekontamination des Zapfluftsystems 20 einbezogen werden, wenn der Primärwärmetauscher 32 ebenfalls dekontaminiert werden soll und/oder wenn dem stromabwärts des Primärwärmetauschers 32 angeordneten Kompressor 36 Luft mit einer erhöhten Temperatur zugeführt werden soll. Um den Primärwärmetauscher 32 in den Prozess zur Dekontamination des Zapfluftsystems 20 einzubeziehen, wird unter der Steuerung des Pack-Steuergeräts 16 das stromaufwärts des Primärwärmetauschers 32 in der Zapfluftleitung 24 angeordnete Zapfluft-Steuerventil 30 geöffnet. Wenn der Primärwärmetauscher 32 dagegen von dem Prozess zur Dekontamination des Zapfluftsystems 20 ausgenommen werden soll, verbleibt das Zapfluft-Steuerventil 30 unter der Steuerung des Pack-Steuergeräts 16 geschlossen.

Wenn eine Dekontamination des Kompressorbereichs 34 des Luftverteilungssystems durchgeführt werden soll, wählt die Wartungscrew in dem Auswahlmenü des Wartungssteuergeräts 100 einen entsprechenden Menüpunkt "Dekontamination Kompressorbereich", woraufhin das Wartungssteuergerät 100 ein Kompressorbereich-Dekontaminations-Aktivierungssignal an das Steuersystem 12 der Flugzeugklimaanlage 10 ausgibt. Die Ausgabe des Kompressorbereich-Dekontaminations-Aktivierungssignals erfolgt an das zentrale Zonen-Steuergerät 18, das seinerseits das Signal an eines der Pack-Steuergeräte 16 oder beide Pack-Steuergeräte 16 weiterleitet.

In Reaktion auf den Empfang des Kompressorbereich-Dekontaminations-Aktivierungssignals steuert das Steuersystem 12 der Flugzeugklimaanlage 10, d. h. eines der Pack-Steuergeräte 16 oder beide Pack-Steuergeräte 16, den Betrieb des Kompressors 36 dann derart, dass der Kompressorbereich 34 auf eine Dekontaminationstemperatur aufgeheizt wird, die höher ist als die Betriebstemperatur des Kompressorbereichs 34 im Normalbetrieb der Flugzeugklimaanlage 10. Insbesondere wird der Kompressorbereich 34 auf eine Dekontaminationstemperatur von ca. 210 bis 230 °C, vorzugsweise von ca. 220 °C aufgeheizt. Im Normalbetrieb der Flugzeugklimaanlage 10 wird der Kompressorbereich 34 dagegen in der Regel bei einer Temperatur von ca. 185 °C betrieben. Das Aufheizen des Kompressorbereichs 34 auf die Dekontaminationstemperatur erfolgt durch eine Erhöhung der Betriebstemperatur des Kompressors 36, die ihrerseits durch eine entsprechende Steuerung einer Staulufteinlassklappe in eine weiter geschlossene Stellung als im Normalbetrieb der Flugzeugklimaanlage 10 erreicht wird.

Für den Fall, dass es unter bestimmten Umgebungsbedingungen, beispielsweise bei kalten Umgebungstemperaturen nicht möglich ist, den Kompressor 36 auf die gewünschte erhöhte Temperatur aufzuheizen, die zur Dekontamination des Kompressorbereichs 84 erforderlich ist, wählt die Wartungscrew in dem Auswahlmenü des Wartungssteuergeräts 100 einen entsprechenden Menüpunkt "Dekontamination Zapfluftsystem + Kompressorbereich", woraufhin das Wartungssteuergerät 100 ein Zapfluftsystem-Dekontaminations-Aktivierungssignal und ein Kompressorbereich-Dekontaminations-Aktivierungssignal an das Steuersystem 12 der Flugzeugklimaanlage 10 ausgibt. Ferner wird unter der Steuerung des Pack-Steuergeräts 16 das stromaufwärts des Primärwärmetauschers 32 in der Zapfluftleitung 24 angeordnete Zapfluft-Steuerventil 30 geöffnet.

Infolgedessen wird dem Kompressor 36 Luft mit einer erhöhten Temperatur zugeführt, sodass es einfacher ist, den Kompressor 36 auf die gewünschte erhöhte Dekontaminationstemperatur aufzuheizen. Ferner wird bei einer derartigen Steuerung der Flugzeugklimaanlage 10 der stromaufwärts des Kompressors 36 angeordnete Primärwärmetauscher 32 ebenfalls in den Dekontaminationsprozess einbezogen.

Wenn eine Dekontamination des Klimatisationsluftzufuhrsystems 48 des Luftverteilungssystems durchgeführt werden soll, wählt die Wartungscrew in dem Auswahlmenü des Wartungssteuergeräts 100 einen entsprechenden Menüpunkt "Dekontamination Klimatisationsluftzufuhrsystem", woraufhin das Wartungssteuergerät 100 ein Klimatisationsluftzufuhrsystem-Dekontaminations-Aktivierungssignal an das Steuersystem 12 der Flugzeugklimaanlage 10 ausgibt. Die Ausgabe des Klimatisationsluftzufuhrsystem-Dekontaminations-Aktivierungssignals erfolgt an das zentrale Zonen-Steuergerät 18.

In Reaktion auf den Empfang des Klimatisationsluftzufuhrsystem-Dekontaminations-Aktivierungssignals steuert das Steuersystem 12 der Flugzeugklimaanlage 10, d. h. das zentrale Zonen-Steuergerät 18 und/oder mindestens eines der Pack-Steuergeräte 16 den Betrieb mindestens eines mit dem Klimatisationsluftzufuhrsystem 48 verbundenen Klimapacks 26, den Betrieb der Trimmluftventile 58 und/oder den Betrieb mindestens eines Rezirkulationsluftgebläses 56 derart, dass das Klimatisationsluftzufuhrsystem 48 auf eine Dekontaminationstemperatur aufgeheizt wird, die höher ist als die Betriebstemperatur des Klimatisationsluftzufuhrsystems 48 im Normalbetrieb der Flugzeugklimaanlage 10. Die Dekontaminationstemperatur in dem Klimatisationsluftzufuhrsystem 48 wird in Abhängigkeit einer zulässigen Maximaltemperatur von in einen Avionikbereich 50a der Flugzeugkabine 52 geleiteter Klimatisationsluft bestimmt. Insbesondere wird das Klimatisationsluftzufuhrsystem 48 auf eine Dekontaminationstemperatur von ca. 60 bis 80 °C, vorzugsweise von ca. 70 °C aufgeheizt.

Das Aufheizen des Klimatisationsluftzufuhrsystems 48 auf die Dekontaminationstemperatur erfolgt durch eine Erhöhung der Temperatur der Luft am Auslass eines oder beider Klimapacks 26 vor dem Eintritt in das Klimatisationsluftzufuhrsystem 48 unter der Steuerung eines Pack-Steuergeräts 16 oder beider Pack-Steuergeräte 16, die Zufuhr von warmer Trimluft durch Öffnen der Trimluftventile 58 unter der Steuerung des zentralen Zonen-Steuergeräts 18 und/oder einen entsprechenden Betrieb der Rezirkulationsgebläses 56 unter der Steuerung des zentralen Zonen-Steuergeräts 18.

## Patentansprüche

1. Verfahren zur Wartung einer Flugzeugklimaanlage (10) mit den Schritten:
- Verbinden eines Wartungssteuergeräts (100) mit einem Steuersystem (12) der Flugzeugklimaanlage (10); und
- Übertragen eines von dem Wartungssteuergerät (100) ausgegebenen Dekontaminations-Aktivierungssignals zur Aktivierung eines Dekontaminationsprozesses an das Steuersystem (12) der Flugzeugklimaanlage (10),
wobei das Steuersystem (12) der Flugzeugklimaanlage (10) in Reaktion auf den Empfang des Dekontaminations-Aktivierungssignals den Betrieb der Flugzeugklimaanlage (10) derart steuert, dass zumindest ein zu dekontaminierender Teilbereich eines Luftverteilungssystems (20, 34, 48) eines mit der Flugzeugklimaanlage (10) ausgestatteten Flugzeugs auf eine Dekontaminationstemperatur aufgeheizt wird, die höher ist als eine Betriebstemperatur des Teilbereichs des Luftverteilungssystems (20, 34, 48) im Normalbetrieb der Flugzeugklimaanlage (10),
**dadurch gekennzeichnet, dass** das Wartungssteuergerät (100):
- zur Aktivierung einer Dekontamination eines Zapfluftsystems (20) des Luftverteilungssystems (20, 34, 48) ein Zapfluftsystem-Dekontaminations-Aktivierungssignal an das Steuersystem (12) der Flugzeugklimaanlage (10) ausgibt und wobei das Steuersystem (12) der Flugzeugklimaanlage (10) in Reaktion auf den Empfang des Zapfluftsystem-Dekontaminations-Aktivierungssignals den Betrieb des Zapfluftsystems (20) der Flugzeugklimaanlage (10) derart steuert, dass das Zapfluftsystem (20) des Luftverteilungssystems (20, 34, 48) auf eine Dekontaminationstemperatur von 220 bis 240 °C aufgeheizt wird, die höher ist als die Betriebstemperatur des Zapfluftsystems (20) im Normalbetrieb der Flugzeugklimaanlage (10); und/oder
- zur Aktivierung einer Dekontamination eines Kompressorbereichs (34) des Luftverteilungssystems (20, 34, 48) ein Kompressorbereich-Dekontaminations-Aktivierungssignal an das Steuersystem (12) der Flugzeugklimaanlage (10) ausgibt und wobei das Steuersystem (12) der Flugzeugklimaanlage (10) in Reaktion auf den Empfang des Kompressorbereich-Dekontaminations-Aktivierungssignals den Betrieb eines Kompressors (36) der Flugzeugklimaanlage (10) derart steuert, dass der Kompressorbereich (34) des Luftverteilungssystems (20, 34, 48) auf eine Dekontaminationstemperatur von 210 bis 230 °C aufgeheizt wird, die höher ist als die Betriebstemperatur des Kompressorbereichs (34) im Normalbetrieb der Flugzeugklimaanlage (10).

2. Verfahren nach Anspruch 1,
wobei
- das Steuersystem (12) der Flugzeugklimaanlage (10) den Betrieb der Flugzeugklimaanlage (10) derart steuert, dass in dem zu dekontaminierenden Teilbereich des Luftverteilungssystems (20, 34, 48) die Dekontaminationstemperatur für eine definierte Zeitspanne beibehalten wird, und/oder
- das Steuersystem (12) der Flugzeugklimaanlage (10) den Betrieb der Flugzeugklimaanlage (10) zur Dekontamination zumindest eines Teilbereichs des Luftverteilungssystems (20, 34, 48) in Abhängigkeit von mindestens einem Umgebungsparameter, insbesondere in Abhängigkeit von einer Umgebungstemperatur steuert.

3. Verfahren nach Anspruch 1 oder 2,
wobei
- die Dekontamination des Zapfluftsystems (20) von einem Zapfluftsystem-Steuergerät (14) des Steuersystems (12) der Flugzeugklimaanlage (10) gesteuert wird, und/oder
- das von dem Wartungssteuergerät (100) ausgegebene Zapfluftsystem-Dekontaminations-Aktivierungssignal von einem zentralen Zonen-Steuergerät (18) des Steuersystems (12) der Flugzeugklimaanlage (10) an das Zapfluftsystem-Steuergerät (14) des Steuersystems (12) der Flugzeugklimaanlage (10) weitergeleitet wird, und/oder
- das Zapfluftsystem (20) des Luftverteilungssystems (20, 34, 48) auf eine Dekontaminationstemperatur von 230 °C aufgeheizt wird, und/oder
- ein mit einer Zapfluftleitung (24) verbundener Primärwärmetauscher (32) der Flugzeugklimaanlage (10), insbesondere durch eine entsprechende Steuerung eines stromaufwärts des Primärwärmetauschers (32) in der Zapfluftleitung (24) angeordneten Zapfluft-Steuerventils (30), wahlweise in einen Prozess zur Dekontamination des Zapfluftsystems (24) einbezogen oder von diesem Prozess ausgenommen wird.

4. Verfahren nach einem der Ansprüche 1 bis 3,
wobei
- die Dekontamination des Kompressorbereichs (34) von einem Pack-Steuergerät (16) des Steuersystems (12) der Flugzeugklimaanlage (10) gesteuert wird, und/oder
- das von dem Wartungssteuergerät (100) ausgegebene Kompressorbereich-Dekontaminations-Aktivierungssignal von dem zentralen Zonen-Steuergerät (18) des Steuersystems (12) der Flugzeugklimaanlage (10) an das Pack-Steuergerät (16) des Steuersystems (12) der Flugzeugklimaanlage (10) weitergeleitet wird, und/oder
- der Kompressorbereich (34) des Luftverteilungssystems (20, 34, 48) auf eine Dekontaminationstemperatur von 220 °C aufgeheizt wird, und/oder
- der Kompressorbereich (34) einen mit einem Auslass des Kompressors (36) verbundenen Hauptwärmetauscher (38) der Flugzeugklimaanlage (10) umfasst.

5. Verfahren nach einem der Ansprüche 1 bis 4,
wobei das Wartungssteuergerät (100) zur Aktivierung einer Dekontamination eines Klimatisationsluftzufuhrsystems (48) des Luftverteilungssystems (20, 34, 48) ein Klimatisationsluftzufuhrsystem-Dekontaminations-Aktivierungssignal an das Steuersystem (12) der Flugzeugklimaanlage (10) ausgibt und wobei das Steuersystem (12) der Flugzeugklimaanlage (10) in Reaktion auf den Empfang des Klimatisationsluftzufuhrsystem-Dekontaminations-Aktivierungssignals den Betrieb eines mit dem Klimatisationsluftzufuhrsystem (48) des Luftverteilungssystems (20, 34, 48) verbundenen Klimapacks (26) der Flugzeugklimaanlage (10), den Betrieb mindestens eines in dem Klimatisationsluftzufuhrsystem (48) des Luftverteilungssystems (20, 34, 48) angeordneten Trimmluftventils (58) und/oder den Betrieb mindestens eines in dem Klimatisationsluftzufuhrsystem (48) des Luftverteilungssystems (20, 34, 48) angeordneten Rezirkulationsluftgebläses (56) derart steuert, dass das Klimatisationsluftzufuhrsystem (48) des Luftverteilungssystems (20, 34, 48) auf eine Dekontaminationstemperatur aufgeheizt wird, die höher ist als die Betriebstemperatur des Klimatisationsluftzufuhrsystems (48) im Normalbetrieb der Flugzeugklimaanlage (10).

6. Verfahren nach Anspruch 5,
wobei
- die Dekontamination des Klimatisationsluftzufuhrsystems (48) von dem Pack-Steuergerät (16) und/oder dem zentralen Zonen-Steuergerät (18) des Steuersystems (12) der Flugzeugklimaanlage (10) gesteuert wird, und/oder
- das von dem Wartungssteuergerät (100) ausgegebene Klimatisationsluftzufuhrsystem-Dekontaminations-Aktivierungssignal von dem zentralen Zonen-Steuergerät (18) des Steuersystems (12) der Flugzeugklimaanlage (10) an das Pack-Steuergerät (16) des Steuersystems (12) der Flugzeugklimaanlage (10) weitergeleitet wird, und/oder
- das Klimatisationsluftzufuhrsystem (48) des Luftverteilungssystems (20, 34, 48) auf eine Dekontaminationstemperatur von 60 bis 80 °C, insbesondere von 70 °C aufgeheizt wird, und/oder
- die Dekontaminationstemperatur in dem Klimatisationsluftzufuhrsystem (48) des Luftverteilungssystems (20, 34, 48) in Abhängigkeit einer zulässigen Maximaltemperatur von in einen Avionikbereich (50a) geleiteter Klimatisationsluft bestimmt wird.

7. Flugzeugklimaanlagensystem (1000), das umfasst:
- eine Flugzeugklimaanlage (10) mit einem Steuersystem (12) und
- ein Wartungssteuergerät (100), das mit dem Steuersystem (12) der Flugzeugklimaanlage (10) verbindbar und dazu eingerichtet ist, ein Dekontaminations-Aktivierungssignal zur Aktivierung eines Dekontaminationsprozesses auszugeben und an das Steuersystem (12) der Flugzeugklimaanlage (10) zu übertragen,
wobei das Steuersystem (12) der Flugzeugklimaanlage (10) dazu eingerichtet ist, in Reaktion auf den Empfang des Dekontaminations-Aktivierungssignals den Betrieb der Flugzeugklimaanlage (10) derart zu steuern, dass zumindest ein zu dekontaminierender Teilbereich eines Luftverteilungssystems (20, 34, 48) eines mit der Flugzeugklimaanlage (10) ausgestatteten Flugzeugs auf eine Dekontaminationstemperatur aufgeheizt wird, die höher ist als eine Betriebstemperatur des Teilbereichs des Luftverteilungssystems (20, 34, 48) im Normalbetrieb der Flugzeugklimaanlage (10), **dadurch gekennzeichnet, dass** das Wartungssteuergerät (100) dazu eingerichtet ist:
- zur Aktivierung einer Dekontamination eines Zapfluftsystems (20) des Luftverteilungssystems (20, 34, 48) ein Zapfluftsystem-Dekontaminations-Aktivierungssignal an das Steuersystem (12) der Flugzeugklimaanlage (10) auszugeben und wobei das Steuersystem (12) der Flugzeugklimaanlage (10) dazu eingerichtet ist, in Reaktion auf den Empfang des Zapfluftsystem-Dekontaminations-Aktivierungssignals den Betrieb des Zapfluftsystems (20) der Flugzeugklimaanlage (10) derart zu steuern, dass das Zapfluftsystem (20) des Luftverteilungssystems (20, 34, 48) auf eine Dekontaminationstemperatur von 220 bis 240 °C aufgeheizt wird, die höher ist als die Betriebstemperatur des Zapfluftsystems (20) im Normalbetrieb der Flugzeugklimaanlage (10), und/oder
- zur Aktivierung einer Dekontamination eines Kompressorbereichs (34) des Luftverteilungssystems (20, 34, 48) ein Kompressorbereich-Dekontaminations-Aktivierungssignal an das Steuersystem (12) der Flugzeugklimaanlage (10) auszugeben und wobei das Steuersystem (12) der Flugzeugklimaanlage (10) dazu eingerichtet ist, in Reaktion auf den Empfang des Kompressorbereich-Dekontaminations-Aktivierungssignals den Betrieb eines Kompressors (36) der Flugzeugklimaanlage (10) derart zu steuern, dass der Kompressorbereich (34) des Luftverteilungssystems (20, 34, 48) auf eine Dekontaminationstemperatur von 210 bis 230 °C aufgeheizt wird, die höher ist als die Betriebstemperatur des Kompressorbereichs (34) im Normalbetrieb der Flugzeugklimaanlage (10).

8. Flugzeugklimaanlagensystem nach Anspruch 7,
wobei
- das Steuersystem (12) der Flugzeugklimaanlage (10) dazu eingerichtet ist, den Betrieb der Flugzeugklimaanlage (10) derart zu steuern, dass in dem zu dekontaminierenden Teilbereich des Luftverteilungssystems (20, 34, 48) die Dekontaminationstemperatur für eine definierte Zeitspanne beibehalten wird, und/oder
- das Steuersystem (12) der Flugzeugklimaanlage (10) dazu eingerichtet ist, den Betrieb der Flugzeugklimaanlage (10) zur Dekontamination zumindest eines Teilbereichs des Luftverteilungssystems (20, 34, 48) in Abhängigkeit von mindestens einem Umgebungsparameter, insbesondere in Abhängigkeit von einer Umgebungstemperatur zu steuern.

9. Flugzeugklimaanlagensystem nach Anspruch 7 oder 8,
wobei das Wartungssteuergerät (100) dazu eingerichtet ist, zur Aktivierung einer Dekontamination eines Klimatisationsluftzufuhrsystems (48) des Luftverteilungssystems (20, 34, 48) ein Klimatisationsluftzufuhrsystem-Dekontaminations-Aktivierungssignal an das Steuersystem (12) der Flugzeugklimaanlage (10) auszugeben und wobei das Steuersystem (12) der Flugzeugklimaanlage (10) dazu eingerichtet ist, in Reaktion auf den Empfang des Klimatisationsluftzufuhrsystem-Dekontaminations-Aktivierungssignals den Betrieb eines mit dem Klimatisationsluftzufuhrsystem (48) des Luftverteilungssystems (20, 34, 48) Klimapacks (26) der Flugzeugklimaanlage (10), den Betrieb mindestens eines in dem Klimatisationsluftzufuhrsystem (48) des Luftverteilungssystems (20, 34, 48) angeordneten Trimmluftventils (58) und/oder den Betrieb mindestens eines in dem Klimatisationsluftzufuhrsystem (48) des Luftverteilungssystems (20, 34, 48) angeordneten Rezirkulationsluftgebläses (56) derart zu steuern, dass das Klimatisationsluftzufuhrsystem (48) des Luftverteilungssystems (20, 34, 48) auf eine Dekontaminationstemperatur aufgeheizt wird, die höher ist als die Betriebstemperatur des Klimatisationsluftzufuhrsystems (48) im Normalbetrieb der Flugzeugklimaanlage (10).

## Claims

1. Method for maintaining an aircraft air-conditioning system (10), comprising the steps of:
- connecting a maintenance control device (100) to a control system (12) of the aircraft air-conditioning system (10); and
- transmitting a decontamination activation signal, output by the maintenance control device (100), for the activation of a decontamination process to the control system (12) of the aircraft air-conditioning system (10),
wherein, in response to receiving the decontamination activation signal, the control system (12) of the aircraft air-conditioning system (10) controls the operation of the aircraft air-conditioning system (10) such that at least a partial region to be decontaminated of an air distribution system (20, 34, 48) of an aircraft equipped with the aircraft air-conditioning system (10) is heated to a decontamination temperature that is higher than an operating temperature of the partial region of the air distribution system (20, 34, 48) during normal operation of the aircraft air-conditioning system (10),
**characterized in that** the maintenance control device (100):
- for activating a decontamination of a bleed air system (20) of the air distribution system (20, 34, 48), outputs a bleed-air-system decontamination activation signal to the control system (12) of the aircraft air-conditioning system (10) and, in response to receiving the bleed-air-system decontamination activation signal, the control system (12) of the aircraft air-conditioning system (10) controls the operation of the bleed air system (20) of the aircraft air-conditioning system (10) such that the bleed air system (20) of the air distribution system (20, 34, 48) is heated to a decontamination temperature of 220 to 240°C, which is higher than the operating temperature of the bleed air system (20) during normal operation of the aircraft air-conditioning system (10); and/or
- for activating a decontamination of a compressor region (34) of the air distribution system (20, 34, 48), outputs a compressor-region decontamination activation signal to the control system (12) of the aircraft air-conditioning system (10) and, in response to receiving the compressor-region decontamination activation signal, the control system (12) of the aircraft air-conditioning system (10) controls the operation of a compressor (36) of the aircraft air-conditioning system (10) such that the compressor region (34) of the air distribution system (20, 34, 48) is heated to a decontamination temperature of 210 to 230°C, which is higher than the operating temperature of the compressor region (34) during normal operation of the aircraft air-conditioning system (10).

2. Method according to Claim 1,
wherein
- the control system (12) of the aircraft air-conditioning system (10) controls the operation of the aircraft air-conditioning system (10) such that the decontamination temperature is maintained for a defined period of time in the partial region to be decontaminated of the air distribution system (20, 34, 48), and/or
- the control system (12) of the aircraft air-conditioning system (10) controls the operation of the aircraft air-conditioning system (10) for the decontamination of at least a partial region of the air distribution system (20, 34, 48) in dependence on at least one environmental parameter, in particular in dependence on an ambient temperature.

3. Method according to Claim 1 or 2,
wherein
- the decontamination of the bleed air system (20) is controlled by a bleed-air-system control device (14) of the control system (12) of the aircraft air-conditioning system (10), and/or
- the bleed-air-system decontamination activation signal output by the maintenance control device (100) is passed on by a central zone control device (18) of the control system (12) of the aircraft air-conditioning system (10) to the bleed-air-system control device (14) of the control system (12) of the aircraft air-conditioning system (10), and/or
- the bleed air system (20) of the air distribution system (20, 34, 48) is heated to a decontamination temperature of 230°C, and/or
- a primary heat exchanger (32), connected to a bleed air line (24), of the aircraft air-conditioning system (10) is selectively included in a process for decontaminating the bleed air system (24) or excluded from this process, in particular by corresponding controlling of a bleed air control valve (30) situated upstream of the primary heat exchanger (32) in the bleed air line (24).

4. Method according to one of Claims 1 to 3,
wherein
- the decontamination of the compressor region (34) is controlled by a pack control device (16) of the control system (12) of the aircraft air-conditioning system (10), and/or
- the compressor-region decontamination activation signal output by the maintenance control device (100) is passed on by the central zone control device (18) of the control system (12) of the aircraft air-conditioning system (10) to the pack control device (16) of the control system (12) of the aircraft air-conditioning system (10), and/or
- the compressor region (34) of the air distribution system (20, 34, 48) is heated to a decontamination temperature of 220°C, and/or
- the compressor region (34) includes a main heat exchanger (38) of the aircraft air-conditioning system (10), connected to an outlet of the compressor (36).

5. Method according to one of Claims 1 to 4,
wherein the maintenance control device (100), for activating a decontamination of an air-conditioning air supply system (48) of the air distribution system (20, 34, 48), outputs an air-conditioning air-supply-system decontamination activation signal to the control system (12) of the aircraft air-conditioning system (10) and, in response to receiving the air-conditioning air-supply-system decontamination activation signal, the control system (12) of the aircraft air-conditioning system (10) controls the operation of an air-conditioning pack (26) of the aircraft air-conditioning system (10) connected to the air-conditioning air supply system (48) of the air distribution system (20, 34, 48), the operation of at least one trim air valve (58) situated in the air-conditioning air supply system (48) of the air distribution system (20, 34, 48) and/or the operation of at least one recirculation air blower (56) situated in the air-conditioning air supply system (48) of the air distribution system (20, 34, 48) such that the air-conditioning air supply system (48) of the air distribution system (20, 34, 48) is heated to a decontamination temperature that is higher than the operating temperature of the air-conditioning air supply system (48) during normal operation of the aircraft air-conditioning system (10).

6. Method according to Claim 5,
wherein
- the decontamination of the air-conditioning air supply system (48) is controlled by the pack control device (16) and/or the central zone control device (18) of the control system (12) of the aircraft air-conditioning system (10), and/or
- the air-conditioning air-supply-system decontamination activation signal output by the maintenance control device (100) is passed on by the central zone control device (18) of the control system (12) of the aircraft air-conditioning system (10) to the pack control device (16) of the control system (12) of the aircraft air-conditioning system (10), and/or
- the air-conditioning air supply system (48) of the air distribution system (20, 34, 48) is heated to a decontamination temperature of 60 to 80°C, in particular of 70°C, and/or
- the decontamination temperature in the air-conditioning air supply system (48) of the air distribution system (20, 34, 48) is determined in dependence on an allowable maximum temperature of air-conditioning air conducted into an avionics region (50a).

7. Aircraft air-conditioning system setup (1000) comprising:
- an aircraft air-conditioning system (10) having a control system (12) and
- a maintenance control device (100), which can be connected to the control system (12) of the aircraft air-conditioning system (10) and is set up to output a decontamination activation signal for the activation of a decontamination process and to transmit it to the control system (12) of the aircraft air-conditioning system (10), wherein the control system (12) of the aircraft air-conditioning system (10) is set up so as to control the operation of the aircraft air-conditioning system (10) in response to receiving the decontamination activation signal such that at least a partial region to be decontaminated of an air distribution system (20, 34, 48) of an aircraft equipped with the aircraft air-conditioning system (10) is heated to a decontamination temperature that is higher than an operating temperature of the partial region of the air distribution system (20, 34, 48) during normal operation of the aircraft air-conditioning system (10), **characterized in that** the maintenance control device (100) is set up:
- for activating a decontamination of a bleed air system (20) of the air distribution system (20, 34, 48), to output a bleed-air-system decontamination activation signal to the control system (12) of the aircraft air-conditioning system (10) and the control system (12) of the aircraft air-conditioning system (10) is set up so as to control the operation of the bleed air system (20) of the aircraft air-conditioning system (10) in response to receiving the bleed-air-system decontamination activation signal such that the bleed air system (20) of the air distribution system (20, 34, 48) is heated to a decontamination temperature of 220 to 240°C, which is higher than the operating temperature of the bleed air system (20) during normal operation of the aircraft air-conditioning system (10), and/or
- for activating a decontamination of a compressor region (34) of the air distribution system (20, 34, 48), to output a compressor-region decontamination activation signal to the control system (12) of the aircraft air-conditioning system (10) and the control system (12) of the aircraft air-conditioning system (10) is set up so as to control the operation of a compressor (36) of the aircraft air-conditioning system (10) in response to receiving the compressor-region decontamination activation signal such that the compressor region (34) of the air distribution system (20, 34, 48) is heated to a decontamination temperature of 210 to 230°C, which is higher than the operating temperature of the compressor region (34) during normal operation of the aircraft air-conditioning system (10).

8. Aircraft air-conditioning system setup according to Claim 7,
wherein
- the control system (12) of the aircraft air-conditioning system (10) is set up so as to control the operation of the aircraft air-conditioning system (10) such that the decontamination temperature is maintained for a defined period of time in the partial region to be decontaminated of the air distribution system (20, 34, 48), and/or
- the control system (12) of the aircraft air-conditioning system (10) is set up so as to control the operation of the aircraft air-conditioning system (10) for the decontamination of at least a partial region of the air distribution system (20, 34, 48) in dependence on at least one environmental parameter, in particular in dependence on an ambient temperature.

9. Aircraft air-conditioning system setup according to Claim 7 or 8,
wherein the maintenance control device (100) is set up so as, for activating a decontamination of an air-conditioning air supply system (48) of the air distribution system (20, 34, 48), to output an air-conditioning air-supply-system decontamination activation signal to the control system (12) of the aircraft air-conditioning system (10) and the control system (12) of the aircraft air-conditioning system (10) is set up so as to control the operation of an air-conditioning pack (26) of the aircraft air-conditioning system (10) to the air-conditioning air supply system (48) of the air distribution system (20, 34, 48), the operation of at least one trim air valve (58) situated in the air-conditioning air supply system (48) of the air distribution system (20, 34, 48) and/or the operation of at least one recirculation air blower (56) situated in the air-conditioning air supply system (48) of the air distribution system (20, 34, 48) in response to receiving the air-conditioning air-supply-system decontamination activation signal such that the air-conditioning air supply system (48) of the air distribution system (20, 34, 48) is heated to a decontamination temperature that is higher than the operating temperature of the air-conditioning air supply system (48) during normal operation of the aircraft air-conditioning system (10).

## Revendications

1. Procédé d'entretien d'une installation (10) de climatisation d'aéronef, comprenant les étapes suivantes :
- connecter un appareil (100) de commande d'entretien à un système (12) de commande de l'installation (10) de climatisation d'aéronef ; et
- transmettre au système (12) de commande de l'installation (10) de climatisation d'aéronef un signal d'activation de décontamination émis par l'appareil (100) de commande d'entretien, pour activer un processus de décontamination,
le système (12) de commande de l'installation (10) de climatisation d'aéronef, en réponse à la réception du signal d'activation de décontamination, commandant le fonctionnement de l'installation (10) de climatisation d'aéronef de telle sorte qu'au moins une zone partielle à décontaminer d'un système (20, 34, 48) de distribution d'air d'un aéronef équipé de l'installation (10) de climatisation d'aéronef soit chauffée à une température de décontamination qui est supérieure à une température de fonctionnement de la zone partielle du système (20, 34, 48) de distribution d'air en fonctionnement normal de l'installation (10) de climatisation d'aéronef,
**caractérisé en ce que** l'appareil (100) de commande d'entretien :
- émet, pour activer une décontamination d'un système (20) de prélèvement d'air du système (20, 34, 48) de distribution d'air, un signal d'activation de décontamination de système de prélèvement d'air à destination du système (12) de commande de l'installation (10) de climatisation d'aéronef, et le système (12) de commande de l'installation (10) de climatisation d'aéronef, en réponse à la réception du signal d'activation de décontamination de système de prélèvement d'air, commandant le fonctionnement du système (20) de prélèvement d'air de l'installation (10) de climatisation d'aéronef de telle sorte que le système (20) de prélèvement d'air du système (20, 34, 48) de distribution d'air soit chauffé à une température de décontamination de 220 à 240°C qui est supérieure à la température de fonctionnement du système (20) de prélèvement d'air pendant le fonctionnement normal de l'installation (10) de climatisation d'aéronef ; et/ou
- émet, pour activer une décontamination d'une zone de compresseur (34) du système (20, 34, 48) de distribution d'air, un signal d'activation de décontamination de zone de compresseur à destination du système (12) de commande de l'installation (10) de climatisation d'aéronef, et le système (12) de commande de l'installation (10) de climatisation d'aéronef, en réponse à la réception du signal d'activation de décontamination de zone de compresseur, commandant le fonctionnement d'un compresseur (36) de l'installation (10) de climatisation d'aéronef de telle sorte que la zone de compresseur (34) du système (20, 34, 48) de distribution d'air est chauffée à une température de décontamination de 210 à 230°C, qui est supérieure à la température de fonctionnement de la zone de compresseur (34) pendant le fonctionnement normal de l'installation (10) de climatisation d'aéronef.

2. Procédé selon la revendication 1,
dans lequel
- le système (12) de commande de l'installation (10) de climatisation d'aéronef commande le fonctionnement de l'installation (10) de climatisation d'aéronef de telle sorte que, dans la zone partielle à décontaminer du système (20, 34, 48) de distribution d'air, la température de décontamination soit maintenue pendant une durée définie, et/ou
- le système de commande (12) de l'installation (10) de climatisation d'aéronef commande le fonctionnement de l'installation (10) de climatisation d'aéronef pour la décontamination d'au moins une zone partielle du système (20, 34, 48) de distribution d'air en fonction d'au moins un paramètre environnemental, notamment en fonction d'une température ambiante.

3. Procédé selon la revendication 1 ou la revendication 2,
dans lequel
- la décontamination du système (20) de prélèvement d'air est commandée par un appareil (14) de commande de système de prélèvement d'air du système (12) de commande du système de climatisation (10) de l'aéronef, et/ou
- le signal d'activation de la décontamination du système de prélèvement d'air, émis par l'appareil (100) de commande d'entretien, est transmis par un appareil (18) de commande de zone centrale du système (12) de commande de l'installation (10) de climatisation d'aéronef à l'appareil (14) de commande du système de prélèvement d'air du système (12) de commande de l'installation (10) de climatisation d'aéronef, et/ou
- le système (20) de prélèvement d'air du système (20, 34, 48) de distribution d'air est chauffé à une température de décontamination de 230°C, et/ou
- un échangeur de chaleur primaire (32) de l'installation (10) de climatisation d'aéronef relié à une conduite de prélèvement d'air (24), en particulier par une commande correspondante d'une vanne (30) de commande de prélèvement d'air agencée en amont de l'échangeur de chaleur primaire (32) dans la conduite de prélèvement d'air (24), est, au choix, inclus dans un processus de décontamination du système de prélèvement d'air (24) ou exclu de ce processus.

4. Procédé selon l'une des revendications 1 à 3,
dans lequel
- la décontamination de la zone de compresseur (34) est commandée par un appareil (16) de commande de bloc du système (12) de commande du système de climatisation de l'aéronef (10), et/ou
- le signal d'activation de décontamination de zone de compresseur émis par l'appareil (100) de commande d'entretien est transmis par l'appareil (18) de commande de zone centrale du système (12) de commande de l'installation (10) de climatisation d'aéronef à l'appareil (16) de commande de bloc du système (12) de commande de l'installation (10) de climatisation d'aéronef, et/ou
- la zone de compresseur (34) du système (20, 34, 48) de distribution d'air est chauffée à une température de décontamination de 220°C, et/ou
- la zone de compresseur (34) comprend un échangeur de chaleur principal (38) de l'installation (10) de climatisation d'aéronef relié à une sortie du compresseur (36).

5. Procédé selon l'une des revendications 1 à 4,
dans lequel l'appareil (100) de commande d'entretien, pour activer une décontamination d'un système (48) d'alimentation en air de climatisation du système (20, 34, 48) de distribution d'air, émet un signal d'activation de décontamination du système d'alimentation en air de climatisation à destination du système (12) de commande de l'installation (10) de climatisation d'aéronef, et dans lequel le système (12) de commande de l'installation (10) de climatisation d'aéronef, en réponse à la réception du signal d'activation de décontamination du système d'alimentation en air de climatisation, commande le fonctionnement d'un bloc de climatisation (26) de l'installation (10) de climatisation d'aéronef connecté au système (48) d'alimentation en air de climatisation du système (20, 34, 48) de distribution d'air, le fonctionnement d'au moins une vanne d'air de compensation (58) agencée dans le système (48) d'alimentation en air de climatisation du système (20, 34, 48) de distribution d'air et/ou le fonctionnement d'au moins un ventilateur d'air de recirculation (56) agencé dans le système (48) d'alimentation en air de climatisation du système (20, 34, 48) de distribution d'air de telle sorte que le système (48) d'alimentation en air de climatisation du système (20, 34, 48) de distribution d'air soit chauffé à une température de décontamination qui est supérieure à la température de fonctionnement du système (48) d'alimentation en air de climatisation en fonctionnement normal de l'installation (10) de climatisation d'aéronef.

6. Procédé selon la revendication 5,
dans lequel
- la décontamination du système (48) d'alimentation en air de climatisation est commandée par l'appareil (16) de commande de bloc et/ou l'unité de commande de zone centrale (18) du système (12) de commande de l'installation (10) de climatisation d'aéronef, et/ou
- le signal d'activation de décontamination du système d'alimentation en air de climatisation émis par l'appareil (100) de commande d'entretien est transmis par l'appareil (18) de commande de zone centrale du système (12) de commande de l'installation (10) de climatisation d'aéronef à l'appareil (16) de commande de bloc du système (12) de commande de l'installation (10) de climatisation d'aéronef, et/ou
- le système (48) d'alimentation en air de climatisation du système (20, 34, 48) de distribution d'air est chauffé à une température de décontamination de 60 à 80°C, en particulier de 70°C, et/ou
- la température de décontamination dans le système (48) d'alimentation en air de climatisation du système (20, 34, 48) de distribution d'air est déterminée en fonction d'une température maximale admissible de l'air de climatisation dirigé vers une zone avionique (50a).

7. Système de climatisation d'aéronef (1000) comprenant :
- une installation (10) de climatisation d'aéronef avec un système de commande (12), et
- un appareil (100) de commande d'entretien, apte à être connecté au système (12) de commande de l'installation (10) de climatisation d'aéronef et adapté pour émettre un signal d'activation de décontamination pour activer un processus de décontamination, et le transmettre au système (12) de commande de l'installation (10) de climatisation d'aéronef,
dans lequel le système (12) de commande de l'installation (10) de climatisation d'aéronef est conçu pour commander, en réponse à la réception du signal d'activation de décontamination, le fonctionnement de l'installation (10) de climatisation d'aéronef de telle sorte qu'au moins une zone partielle à décontaminer d'un système (20, 34, 48) de distribution d'air d'un aéronef équipé de l'installation (10) de climatisation d'aéronef est chauffée à une température de décontamination qui est supérieure à une température de fonctionnement de la zone partielle du système (20, 34, 48) de distribution d'air en fonctionnement normal de l'installation (10) de climatisation d'aéronef, **caractérisé en ce que** l'appareil (100) de commande d'entretien est conçu de façon à :
- émettre, pour activer une décontamination d'un système (20) de prélèvement d'air du système (20, 34, 48) de distribution d'air, un signal d'activation de décontamination de système de prélèvement d'air à destination du système (12) de commande de l'installation (10) de climatisation d'aéronef, et le système (12) de commande de l'installation (10) de climatisation d'aéronef étant conçu pour, en réponse à la réception du signal d'activation de décontamination du système de distribution d'air, commander le fonctionnement du système (20) de prélèvement d'air de l'installation (10) de climatisation d'aéronef de telle sorte que le système (20) de prélèvement d'air du système (20, 34, 48) de distribution d'air soit chauffé à une température de décontamination de 220 à 240°C, qui est supérieure à la température de fonctionnement du système (20) de prélèvement d'air pendant le fonctionnement normal de l'installation (10) de climatisation d'aéronef, et/ou
- émettre, pour activer une décontamination d'une zone de compresseur (34) du système (20, 34, 48) de distribution d'air, un signal d'activation de décontamination de zone de compresseur à destination du système (12) de commande de l'installation (10) de climatisation d'aéronef, et le système (12) de commande de l'installation (10) de climatisation d'aéronef étant conçu pour, en réponse à la réception du signal d'activation de décontamination de zone de compresseur, commander le fonctionnement d'un compresseur (36) de l'installation (10) de climatisation d'aéronef de telle sorte que la zone de compresseur (34) du système (20, 34, 48) de distribution d'air soit chauffée à une température de décontamination de 210 à 230°C, qui est supérieure à la température de fonctionnement de la zone de compresseur (34) en fonctionnement normal de l'installation (10) de climatisation d'aéronef.

8. Système de climatisation d'aéronef selon la revendication 7,
dans lequel
- le système de commande (12) de l'installation (10) de climatisation d'aéronef est conçu pour commander le fonctionnement de l'installation (10) de climatisation d'aéronef de telle sorte que, dans la zone partielle à décontaminer du système (20, 34, 48) de distribution d'air, la température de décontamination soit maintenue pendant une durée définie, et/ou
- le système (12) de commande de l'installation (10) de climatisation d'aéronef est conçu pour commander le fonctionnement de l'installation (10) de climatisation d'aéronef pour la décontamination d'au moins une zone partielle du système (20, 34, 48) de distribution d'air en fonction d'au moins un paramètre environnemental, notamment en fonction d'une température ambiante.

9. Système de climatisation d'aéronef selon la revendication 7 ou la revendication 8,
dans lequel l'appareil (100) de commande d'entretien est conçu pour émettre un signal d'activation de décontamination de système d'alimentation en air de climatisation à destination du système (12) de commande de l'installation (10) de climatisation d'aéronef pour activer une décontamination du système (48) d'alimentation en air de climatisation du système (20, 34, 48) de distribution d'air, et dans lequel le système (12) de commande de l'installation (10) de climatisation d'aéronef est conçu pour, en réponse à la réception du signal d'activation de décontamination du système d'alimentation en air de climatisation, commander le fonctionnement d'un bloc de climatisation (26) de l'installation (10) de climatisation d'aéronef au système (48) d'alimentation en air de climatisation du système (20, 34, 48) de distribution d'air, le fonctionnement d'au moins une vanne d'équilibrage (58) agencée dans le système (48) d'alimentation en air de climatisation du système (20, 34, 48) de distribution d'air et/ou le fonctionnement d'au moins un ventilateur d'air de recirculation (56) agencé dans le système (48) d'alimentation en air de climatisation du système (20, 34, 48) de distribution d'air, de telle sorte que le système (48) d'alimentation en air de climatisation du système (20, 34, 48) de distribution d'air soit chauffé à une température de décontamination qui est supérieure à la température de fonctionnement du système (48) d'alimentation en air de climatisation en fonctionnement normal de l'installation (10) de climatisation d'aé
